# EUROPEAN PATENT APPLICATION

(11) **EP 3 467 093 A1**
(43) Date of publication of application: **10.04.2019**
(21) Application number: 17802382.6
(22) Date of filing: 01.03.2017
(51) Int. Cl.: C12M 1/42, C12N 5/00

(54) **DEVICE AND METHOD FOR FABRICATING THREE-DIMENSIONAL STRUCTURE FROM CELLS**

(30) Priority: 27.05.2016 JP 2016105762
(71) Applicant: Hitachi High-Technologies Corporation, Tokyo 105-8717 (JP)
(72) Inventor: KAWACHI Ryosuke, Tokyo 105-8717 (JP); SAKURAI Toshinari, Tokyo 105-8717 (JP)
(74) Representative: Strehl Schübel-Hopf & Partner
(86) International application number: PCT/JP2017/008048
(87) International publication number: WO 2017/203780

(57) **Abstract**

There is a need for fabrication of a three-dimensional structure for forming a regenerated tissue/regenerated organ having a desired size, shape, or structure by adjusting culture conditions with high precision and without causing contamination or damage to cells. Provided are: a device for acquiring a fabrication condition of a three-dimensional structure to be fabricated on the basis of externally inputted information relating to the three-dimensional structure, generating a magnetic field in a culture container for cells on the basis of the acquired fabrication condition, supplying magnetized cells to the culture container in which the magnetic field is generated, and culturing the supplied cells; and a method using the device.

## Description

### Technical Field

The present invention relates to a technique for fabricating a three-dimensional structure from cells, and more particularly, to a device and a method for fabricating a three-dimensional structure having an arbitrary shape such as a cell mass by performing cell manipulation to cells such as spheroid cells incorporating a magnetic substance therein by using an external magnetic field.

### Background Art

In recent years, with the development of regenerative medical technology, regenerated tissue and regenerated organ are demanded not only for research use, but also for therapeutic use in clinical practice.

PTL 1 discloses a technique for manipulating cells (such as spheroid cells) and forming a cell mass having an arbitrary shape by culturing the spheroid cells in a state where the spheroid cells are skewered on needles in a shape of a pin holder.

PTL 2 discloses a technique for forming a tissue by adhering cells to one another by generating a centrifugal force in a culture vessel for culturing cells or magnetizing cells with magnetic particles and generating a magnetic force from outside by using a magnet.

PTL 3 discloses a technique for forming a multilayered tissue by culturing cells magnetized by magnetic particles in a state where the cells are attached to the bottom surface of a culture vessel by a magnetic force of a magnet.

### Citation List

### Patent Literature

PTL 1: WO 2008/123614
PTL 2: JP-A-2010-161954
PTL 3: WO 2004/083412

### Summary of Invention

### Technical Problem

When forming a regenerated tissue/regenerated organ having a desired size, shape, or structure by using cells collected from a living body, it is necessary to adjust culture conditions with high precision and without causing contamination or damage to cells.

However, with the technique disclosed in PTL 1, during manipulation of cells, in order to form a cell mass having an arbitrary shape, cells are skewered directly to a plurality of needles in a shape of a pin holder. Therefore, damages to cells are not negligible, and there is also a risk of contamination.

With the techniques described in PTL 2 and PTL 3, as described above, adhesion between cells and a culture vessel and adhesion between the cells are promoted by using a centrifugal force or a magnetic force from the outside, it is not necessary to directly contact the cells, and thus influence from contamination and damage can be reduced. However, no specific culturing method for forming a regenerated tissue/regenerated organ with a desired shape or size is taken into consideration.

In view of the above-described problems, the present invention relates to realization of formation of a regenerated tissue/regenerated organ having a desired size, shape, or structure by adjusting culture conditions with high precision and without causing contamination or damage to cells.

### Solution to Problem

According to an aspect of the present invention, there is provided a device and a method using the device. The device includes a culture vessel in which magnetized cells are arranged; a magnetic field generator configured to generate a magnetic field in the culture vessel; an incubator configured to accommodate the culture vessel therein and maintain the interior of the culture vessel at a predetermined temperature; and a control unit configured to control culture conditions of cells arranged in the culture vessel, in which, when information about a three-dimensional structure to be fabricated is input from an operator, the control unit acquires fabrication condition for the corresponding three-dimensional structure based on the input information, and controls the magnetic field generator to generate a magnetic field based on the acquired fabrication conditions.

### Advantageous Effects of Invention

According to the above aspect, a regenerated tissue/regenerated organ having a desired size, shape, or structure can be formed by adjusting culture conditions with high precision and without causing contamination or damage to cells.

### Brief Description of Drawings

Fig. 1 is a diagram illustrating a basic configuration of a device for fabricating a three-dimensional structure from cells according to the present embodiment.
Fig. 2 is a diagram illustrating the basic configuration of a magnetic field generator according to the present embodiment.
Fig. 3 is a flowchart illustrating basic operation for fabricating a three-dimensional structure from cells according to the present embodiment (Embodiment 1).
Fig. 4 is a diagram illustrating an example of a magnetic field formation pattern according to the present embodiment (Embodiment 1).
Fig. 5 is a diagram illustrating an example of an operation for introducing cells into a culture vessel according to the present embodiment (Embodiment 1).
Fig. 6 is a diagram illustrating a state (a) of single layer cells at the beginning of culturing (before fixed) and a state (b) of the single layer cells after culturing (fixed) according to the present embodiment (Embodiment 1).
Fig. 7 is a diagram illustrating a process for making multilayered cells according to the present embodiment (Embodiment 1).
Fig. 8 is a flowchart illustrating basic operation for fabricating a three-dimensional structure from cells according to the present embodiment (Embodiment 2).
Fig. 9 is a diagram illustrating an operation for introducing cells into a culture vessel according to the present embodiment (Embodiment 2).
Fig. 10 is a diagram illustrating an example of checking locations of cells by using a camera according to the present embodiment (Embodiment 2).
Fig. 11 is a diagram illustrating a specific example of a magnetic field formation pattern according to the present embodiment (Embodiment 2).
Fig. 12 is a diagram illustrating a state (a) of single layer or multilayer cells at the beginning of culturing (before fixed) and a state (b) of the single layer or multilayer cells after culturing (fixed) according to the present embodiment (Embodiment 2).
Fig. 13 is a flowchart illustrating basic operation for fabricating a three-dimensional structure from a plurality of cells with different diameters according to the present embodiment (Embodiment 3).
Fig. 14 is a diagram illustrating states from the beginning of culturing to fixation of cells according to the present embodiment (Embodiment 3).
Fig. 15 is a flowchart illustrating basic operation for fabricating a three-dimensional structure from cells, including a step of removing magnetic beads according to the present embodiment (Embodiment 4).
Fig. 16 is a flowchart illustrating basic operation for fabricating a three-dimensional structure from cells, including a step of removing magnetic beads according to the present embodiment (Embodiment 4).
Fig. 17 is a flowchart illustrating details of a step of removing magnetic beads according to the present embodiment (Embodiment 4).
Fig. 18 is a functional block diagram of determining fabrication conditions for a three-dimensional structure from cells according to the present embodiment.
Fig. 19 is a diagram illustrating fabrication images of a three-dimensional structure from cells according to the present embodiment.
Fig. 20 is a diagram illustrating a GUI screen displaying information about a three-dimensional structure from cells according to the present embodiment (Embodiment 1 to Embodiment 3).
Fig. 21 is a diagram illustrating a GUI screen displaying information about a three-dimensional structure from cells according to the present embodiment (Embodiment 4) .

### Description of Embodiments

### <Overall Configuration of Device>

Hereinafter, the present embodiment will be described below in detail with reference to the drawings. Throughout the description, redundant explanation may be omitted for each component having the same function in the drawings for describing the present embodiment.

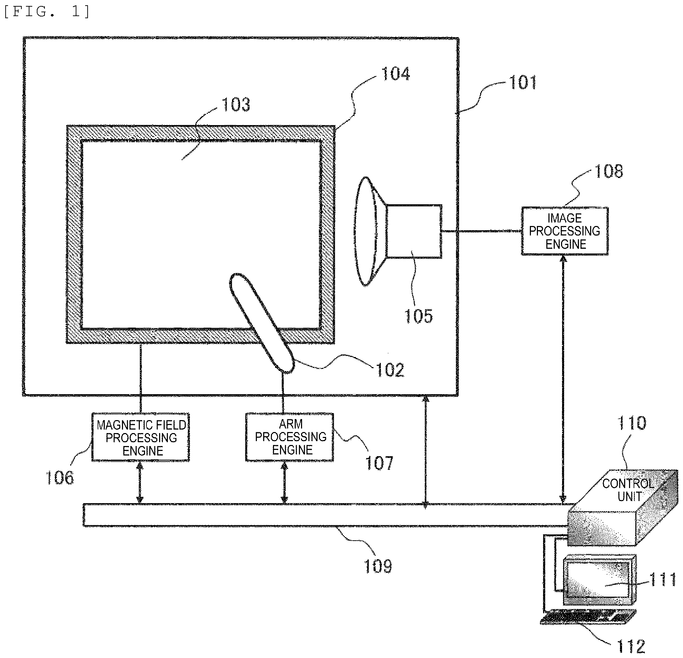

Fig. 1 is a diagram illustrating a basic configuration of a device for fabricating a three-dimensional structure from cells according to the present embodiment.

The device of the present embodiment mainly includes a culture vessel 103, an arm 102, a magnetic field generator 104, and a camera 105 in an incubator 101. The device of the present embodiment further includes a magnetic field processing engine 106, an arm processing engine 107, an image processing engine 108, a data bus 109, a control unit 110, a display unit 111, and an input unit 112 that are outside the incubator 101. The incubator 101 can adjust internal temperature, oxygen concentration, and carbon dioxide concentration therein. In order to capture a clear image, it is desirable for the camera 105 to be installed in the incubator 101. However, when the internal temperature, the oxygen concentration, and the carbon dioxide concentration are inappropriate for capturing an image or in order to reduce the risk of contamination, the camera 105 may be installed outside the incubator 101.

Furthermore, throughout the description, the control unit 110 controls operation or condition setting/adjustment of various components included in the device of the description, such as adjustment of environmental conditions inside the incubator 101, operation of the arm 102, adjustment of magnetic field generation conditions by the magnetic field generator 104, and adjustment of image capturing operation and conditions using the camera 105. The control unit 110 can exchange information with the magnetic field processing engine 106, the arm processing engine 107, and the image processing engine 108 via the data bus 109, thereby transmitting instructions to various components or acquiring data, such as signals and images acquired from various components. In addition, the control unit 110 may acquire and store various types of data related to cell culture conditions and information including instructions input by an operator via the input unit 112 (a keyboard, a mouse, and the like), calculate conditions for fabricating a three-dimensional structure from cells described below, and display the above-stated information on the display unit 111 for displaying a graphical user interface (GUI) or the like. Furthermore, in Fig. 1, the control unit 110 is connected to each component and controls the entire device of the present embodiment. However, each component may include an independent control unit.

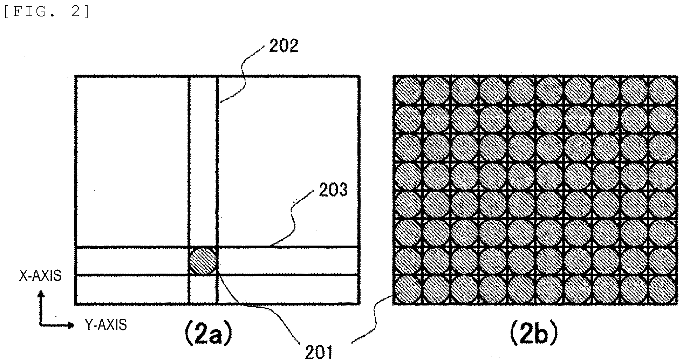

Next, the configuration of the above-stated magnetic field generator 104 will be described below in more detail with reference to Fig. 2.

Fig. 2 is a diagram illustrating the basic configuration of a magnetic field generator according to the present embodiment. Techniques for applying a magnetic field include a technique illustrated in (2a) for applying a magnetic field to a spot at a particular location and a technique illustrated in (2b) for applying a planar magnetic field throughout a predetermined region. In the technique illustrated in (2a), an electromagnet 201 includes an X-axis movement adjusting mechanism 202 and a Y-axis movement adjusting mechanism 203. The position of the electromagnet 201 on the X-Y plane can be freely changed by using the X-axis movement adjusting mechanism 202 and the Y-axis movement adjusting mechanism 203. In the technique illustrated in (2b), a plurality of electromagnets 201 are arranged on the X-Y plane, and thus magnetic force can be applied by the electromagnets 201 arranged in a desired region. Here, the size of the electromagnet 201 is not limited. However, in either the technique illustrated in (2a) or the technique illustrated in (2b), cells magnetized by incorporating a magnetic substance (hereinafter simply referred to as "magnetized cells") are manipulated by using the electromagnet 201. Therefore, in order to improve the positional accuracy during cell manipulation, it is desirable to select small electromagnets 201. In addition, when at least the diameter of the electromagnets 201 is smaller than the diameter of magnetized cells, the positional accuracy during cell manipulation is relatively high and reproducibility of a three-dimensional structure from cells can be improved.

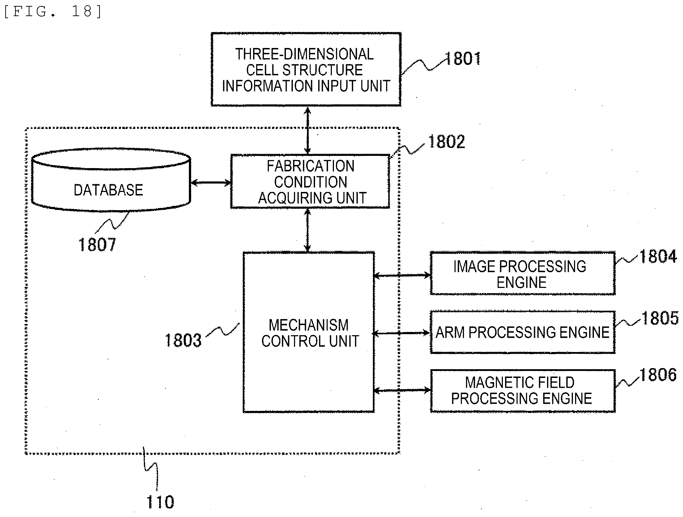

Here, Fig. 18 is a functional block diagram of determining fabrication conditions for a three-dimensional structure from cells according to the present embodiment. When the operator inputs information about a desired three-dimensional structure to be fabricated (shape image, size, thickness, and the like) through a three-dimensional cell structure information input unit 1801 in the input unit 112 illustrated in Fig. 1, a fabrication condition acquiring unit 1802 acquires optimal fabrication conditions for the three-dimensional structure based on the input information and information stored in a database 1807 (various information including characteristics such as adhesion conditions according to types of cells, time restriction of fabrication, types and characteristics of magnetic beads, and the like). Thereafter, a mechanism control unit 1803 transmits instruction signals to an image processing engine 1804, an arm processing engine 1805, and a magnetic field processing engine 1806 in order to start fabrication operation based on the acquired conditions.

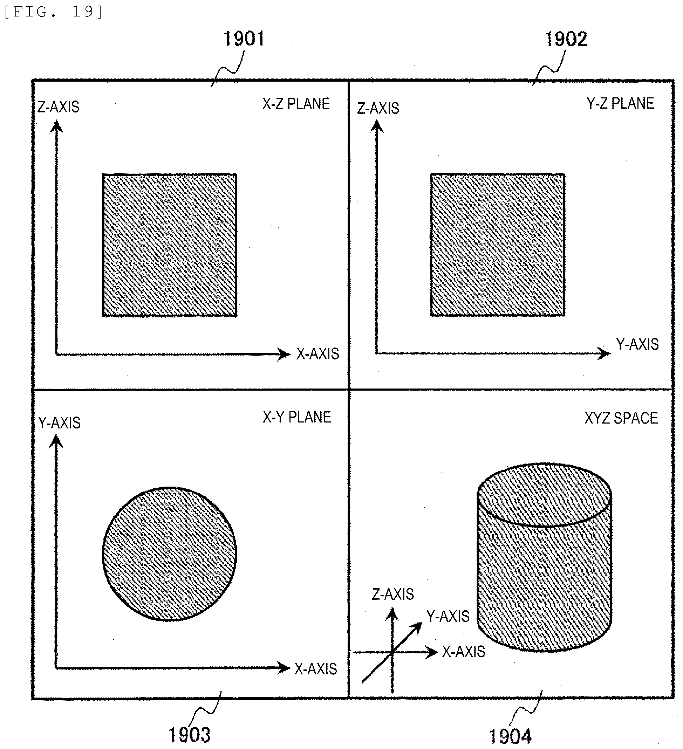

Here, Fig. 19 is a diagram illustrating fabrication images of a three-dimensional structure from cells according to the present embodiment. As illustrated in Fig. 19, the operator can visually confirm images of a three-dimensional structure from cells in an X-Z plane 1901, a Y-Z plane 1902, an X-Y plane 1903, and an XYZ space 1904 displayed on the GUI of the display unit 111. Furthermore, the operator can design an image of a three-dimensional structure with a desired size and a desired thickness by adjusting lengths of horizontal axes and vertical axes in each of the planes 1901 to 1903. Here, in the present embodiment, a case of displaying fabrication images of a three-dimensional structure in all of the X-Z plane 1901, the Y-Z plane 1902, the X-Y plane 1903, and the XYZ space 1904 is described. However, a fabrication image may be selectively displayed among any one of them.

### Embodiment 1

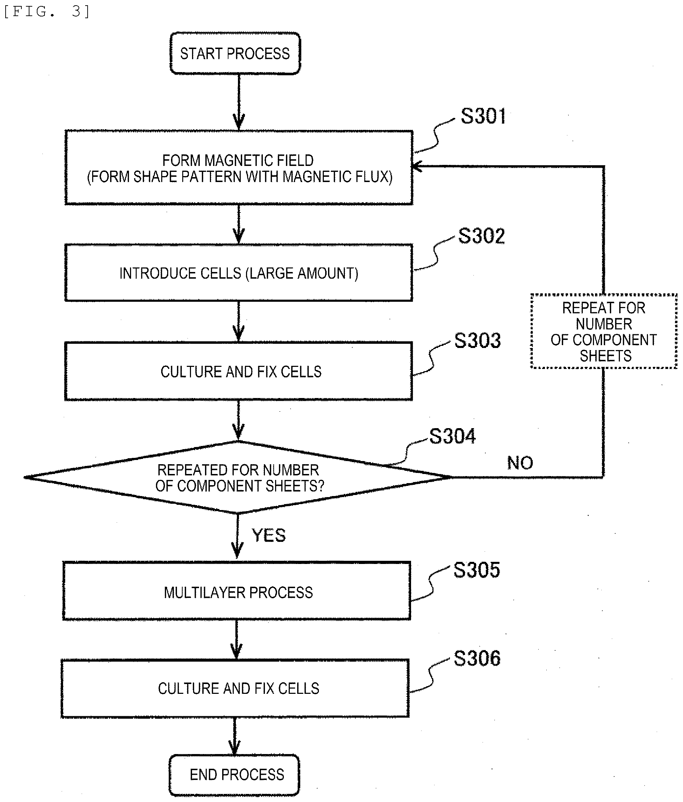

Next, fabrication of a three-dimensional structure from cells using the above-described device will be described below with reference to Figs. 3 to 7 and Fig. 20.

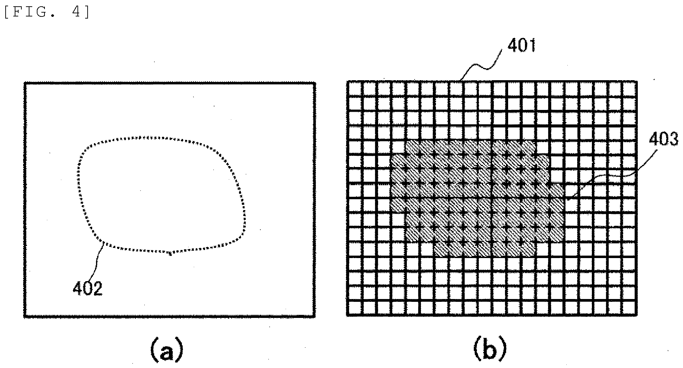

Fig. 3 is a flowchart illustrating basic operation for fabricating a three-dimensional structure cells according to the present embodiment, and Fig. 4 is a diagram illustrating an example of a magnetic field formation pattern according to the present embodiment.

First, in the magnetic field generator 104, based on a target shape (shape pattern) 402 of a cell mass illustrated in Fig. 4, a magnetic field application pattern 403 set similarly to the target shape is applied (step S301). In the present embodiment, the magnetic field generator 104 employs the technique illustrated in (2b) for applying a planar magnetic field throughout a predetermined region.

When applying a magnetic field, the magnetic field is applied only by the electromagnet 201 in a region included in the magnetic field application pattern 403 set similarly to the target shape (shape pattern) 402 of the cell mass through the magnetic field processing engine 106. In this case, the electromagnet 201 is preferably a solenoid coil capable of controlling a magnetic flux perpendicular to a surface with a current value.

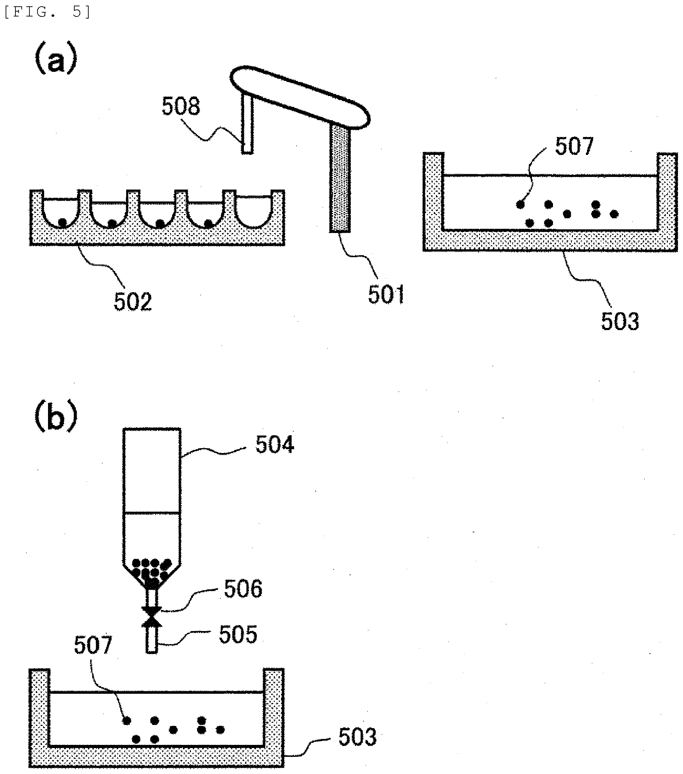

Next, magnetized cells incorporating magnetic substances such as magnetic beads are introduced into the culture vessel 103 (step S302) as much as the number of cells constituting a single layer cell sheet. Here, Fig. 5 illustrates an example of an operation for introducing cells into a culture vessel. Here, as illustrated in Fig. 5(a), a method of introducing individual spheroid cells 507 cultured in a well plate 502 into the culture vessel 503 by using an arm 501 is illustrated. In order to improve the reproducibility of a single layer cell sheet, it is preferable that the cell diameter of the individual spheroid cells 507 cultured in the well plate 502 is as uniform as possible. In this case, the individual spheroid cells 507 cultured in the well plate 502 may be reliably transferred to the culture vessel 503 one by one in order to reduce damages to cells . Furthermore, in order to improve the efficiency of introduction, a plurality of spheroid cells 507 may be introduced into the culture vessel 503 during a single suction operation of a nozzle 508. In step S302, when a magnetic substance is incorporated in the spheroid cell 507, the electromagnet 201 may be installed at the leading end of the nozzle 508, and the spheroid cell 507 may be manipulated by using a magnetic field and introduced into the culture vessel 503. Alternatively, as illustrated in Fig. 5(b), the cultured spheroid cell 507 of the number equal to the number of the cells constituting the single layer cell sheet may be collected in a container like a centrifuge tube 504, and the optimal number of the spheroid cells 507 for forming the target shape 402 of a cell mass may be introduced into the culture vessel 503 by using a tube 505 and a valve 506 provided in the centrifuge tube 504. In this case, in order to complete the introduction within a short period of time, the introduction may be performed after the spheroid cells 507 are condensed at the bottom of the centrifuge tube 504 by centrifuging the centrifuge tube 504 including a large number of spheroid cells 507 or, when a magnetic substance is incorporated in the spheroid cells 507, by using an external magnetic field.

Next, cells introduced into the culture vessel 103 are cultured and fixed (step S303) .

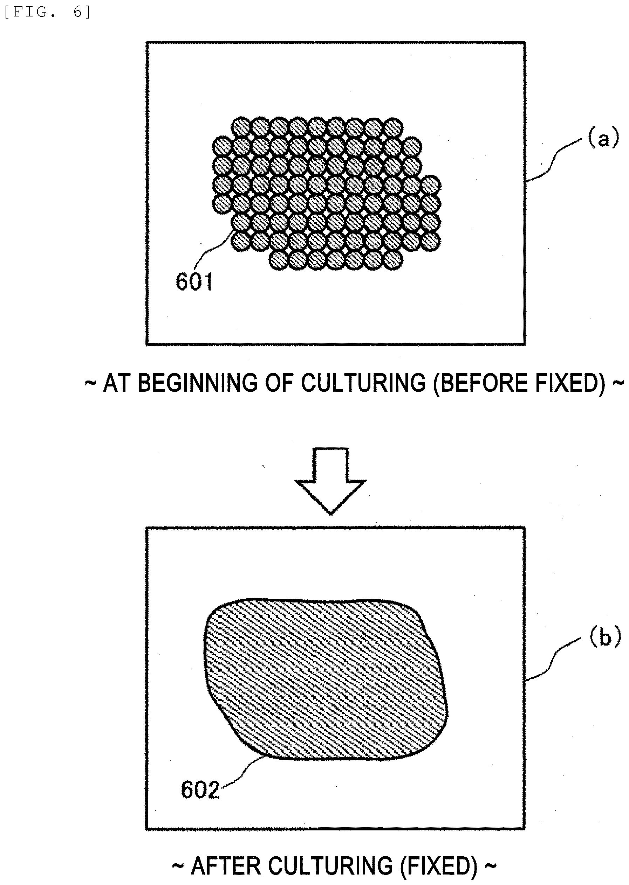

Here, Fig. 6 illustrates a state (a) of single layer cells at the beginning of culturing (before fixed) and a state (b) of the single layer cells after culturing (fixed) according to the present embodiment. According to the magnetic field pattern 403 applied in step S301, cells incorporating a magnetic substance are arranged in a single layer shape. As illustrated in Fig. 6(a), when optimum temperature, oxygen concentration, and carbon dioxide concentration are given within the incubator 101, among cells 601 arranged in a single layer shape, as illustrated in Fig. 6(b), individual spheroid cells are fused with one another and fixed, and thus a single layer cell sheet 602 is formed.

Next, in step S304, it is determined about cell sheets 605 transformed to single layer sheets, whether all of the number of cell sheets necessary for fabricating a target three-dimensional cell mass are formed (step S304). Here, when not all numbers of sheets necessary are formed, steps S301 and S302 are repeated to form the necessary numbers of single layer cell sheets.

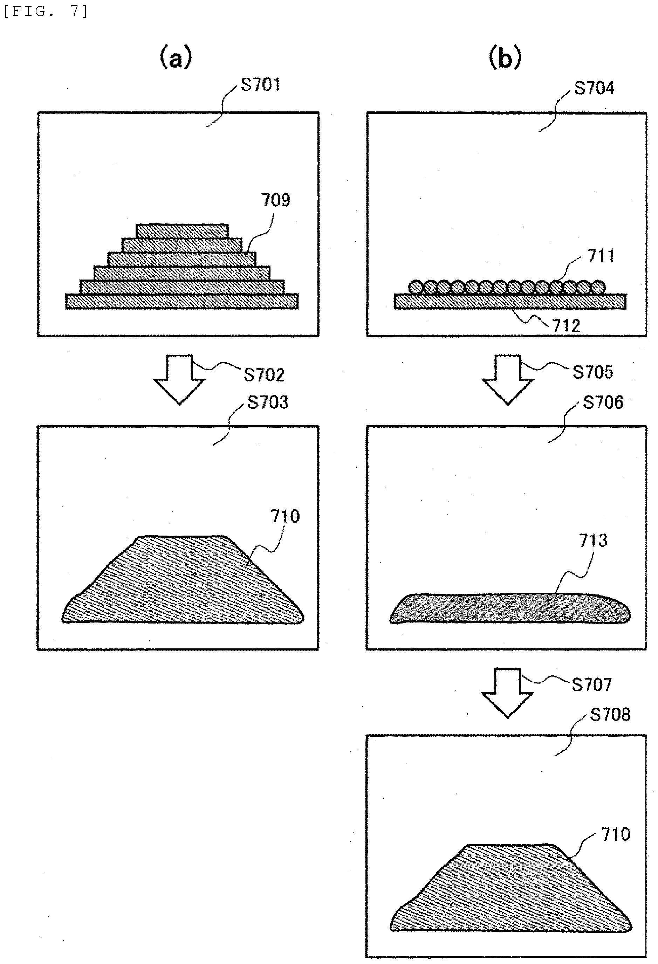

When all the necessary single layer cell sheets are formed, the process proceeds to a multilayer process (step S305) . Here, Fig. 7 illustrates a process for making multilayered cells according to the present embodiment.

As illustrated in Fig. 7 (a), all of the single layer cell sheets formed in step S304 are arranged as multilayer 709 (step S701) . When the single layer cell sheets are cultured by giving optimum temperature, oxygen concentration, and carbon dioxide concentration in the incubator 101 (step S702), individual single layer cell sheets are fused with one another and fixed, and thus a target cell mass 710 is formed (step S703) . Here, a method of the manipulating a multilayer includes stacking the individual single layer cell sheets by using the arm 501. Furthermore, all of the single layer cell sheets formed in step S304 are arranged in respective culture vessels 103 prepared as many as the number of single layer cell sheets or in spaces elsewhere within one culture vessel 103. For this reason, the method of Fig. 7(a) requires the culture vessel 103 having a large area or a large volume, and thus the size of a fabrication device may become inevitably large. Therefore, there is an alternative method illustrated in Fig. 7(b) capable of making a multilayer with the culture vessel 103 having at least an area corresponding to an area of one single layer cell sheet. As illustrated in Fig. 7(b), the magnetic field generator 104 applies a magnetic field pattern for a new layer onto the single layer cell sheet 712 formed in step S303 in the culture vessel 103, and as illustrated in Fig. 5, cells are introduced into the culture vessel 103, thereby arranging a new cell layer having a shape of a single layer cell sheet through a magnetic field (step S704). Next, when cultured in the incubator 101 by giving optimum temperature, oxygen concentration, and carbon dioxide concentration (step S705), a second layer cell sheet is fused and fixed, thereby forming cell mass 713 of two layers (step S706). By repeating steps S704, S705, and S706 described above, a cell mass having the same shape as the cell mass formed using the method illustrated in Fig. 7(a) can be formed. Furthermore, in the method of Fig. 7(b), if there is the culture vessel 103 having at least an area corresponding to the area of one single layer cell sheet, multilayer can be made, and thus the size of a fabrication device can be further reduced.

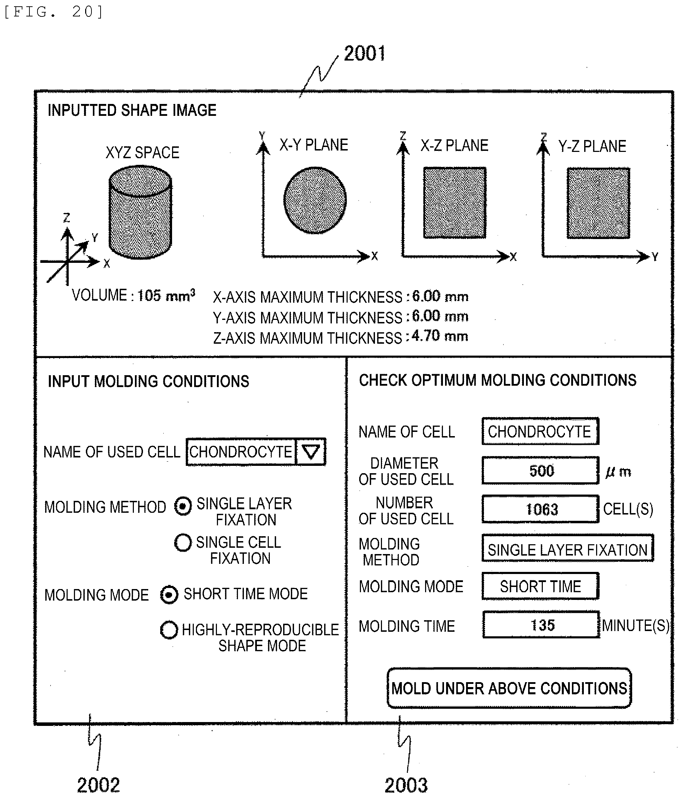

Here, Fig. 20 is a diagram illustrating a GUI screen displaying information about a three-dimensional structure from cells according to the present embodiment. A shape image display unit 2001 displays an image of a three-dimensional structure from cells designed using the method described above with reference to Fig. 19. In addition, the operator can input conditions including a name of used cell, a molding method (a single layer fixation, a single cell fixation, or the like), a molding mode (short time mode, highly-reproducible shape mode, or the like), and the like which are determined based on a purpose of a three-dimensional structure to be fabricated or a tissue to be applied through a molding condition input unit 2002. Then, in an optimum molding condition display unit 2003, the optimum conditions acquired by the fabrication condition acquiring unit 1802 described above with reference to Fig. 18 are displayed and, when starting a molding process under displayed conditions, the operator may instruct to start the molding process through an input button indicated as "mold under above conditions".

Input and confirmation of fabrication conditions of a three-dimensional structure through the GUI screen can also be applied to an Embodiment 2 and an Embodiment 3 described below.

### Embodiment 2

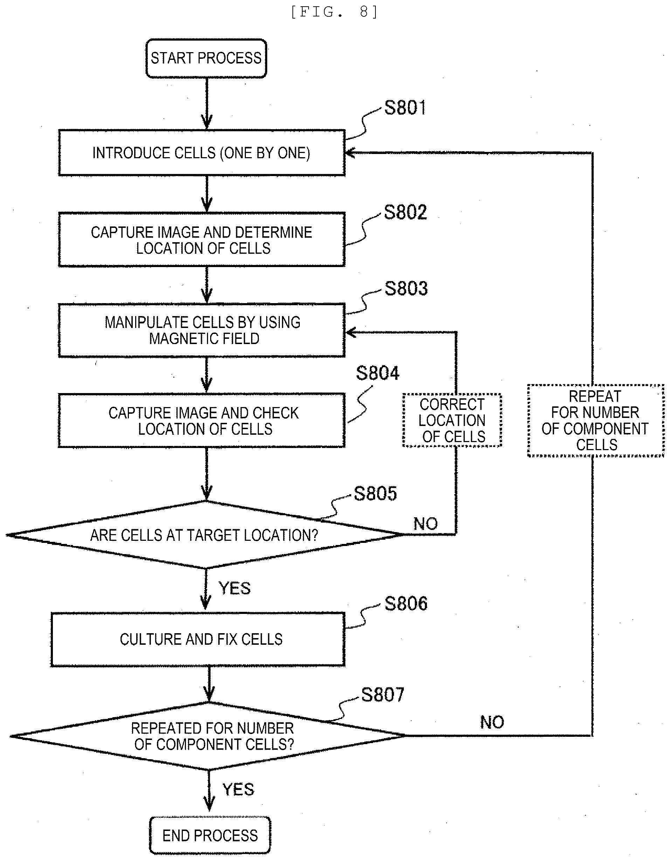

In the Embodiment 1 described above, a case of introducing cells of the number equal to the number of cells constituting a single layer cell sheet into the culture vessel 103 at once (step S302) has been described. Next, in the present embodiment, in order to further improve the reproducibility of a single layer cell sheet, fabrication of a three-dimensional structure from cells using a technique of introducing cells one by one into the culture vessel 103 will be described with reference to Figs. 8 to 12.

Fig. 8 is a flowchart illustrating operations for fabricating a three-dimensional structure from cells according to the present embodiment. First, cells are introduced into the culture vessel 103 one by one (step S801) . At this time, based on information input by an operator as described above, a control unit sets target location in the culture vessel 103 where cells will be arranged and move the cells to the target location in order to fabricate a desired three-dimensional structure. In the present embodiment, the magnetic field generator 104 employs the technique illustrated in (2a) of moving the electromagnet 201 toward a target location by using the X-axis movement adjusting mechanism 202 and the Y-axis movement adjusting mechanism 203 and applying a magnetic field to a particular spot.

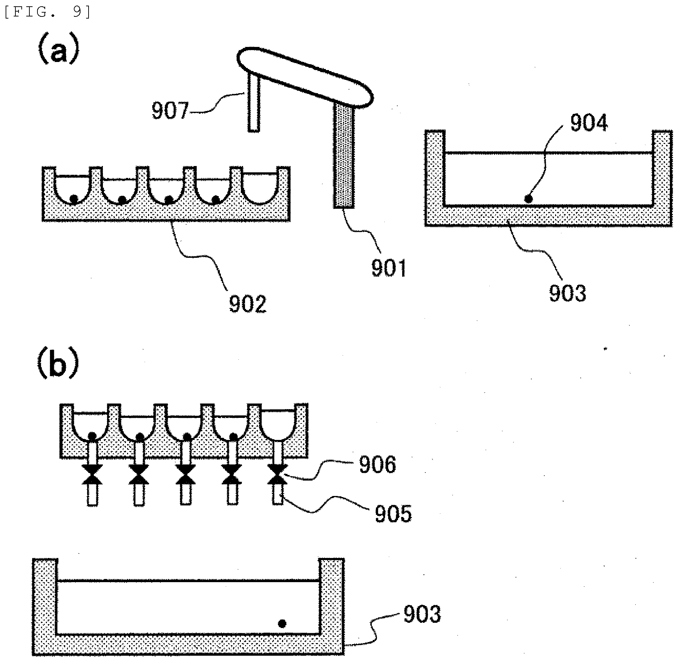

Here, Fig. 9 illustrates an operation for introducing cells into a culture vessel according to the present embodiment. As illustrated in Fig. 9(a), there is a method of introducing each spheroid cell 904 cultured in a well plate 902 into a culture vessel 903 by using an arm 901.

In order to improve the reproducibility of a cell mass, it is preferable that the cell diameter of the individual spheroid cells 904 cultured in the well plate 902 is as uniform as possible. In the case where a magnetic substance is incorporated in the spheroid cell 904 at the time of step S801, an electromagnet may be installed at the leading end of a nozzle 907 and the spheroid cell 904 can be introduced into the culture vessel 903 after manipulating the spheroid cell 904 by using a magnetic field. Alternatively, as illustrated in Fig. 9(b), there is a technique of introducing the individual spheroid cells 904 cultured in the well plate 902 into the culture vessel 903 through a tube 905 and a valve 906 connected to the well plate 902. In this case, after confirming with the camera 105 that introduced cells are fixed at locations (target locations) to be arranged to fabricate a three-dimensional structure, the valve 906 for next cells can be opened for cell introduction. Accordingly, since there are no contact objects such as the arm 901 when cells are introduced, cells can be introduced with a minimum risk of contamination.

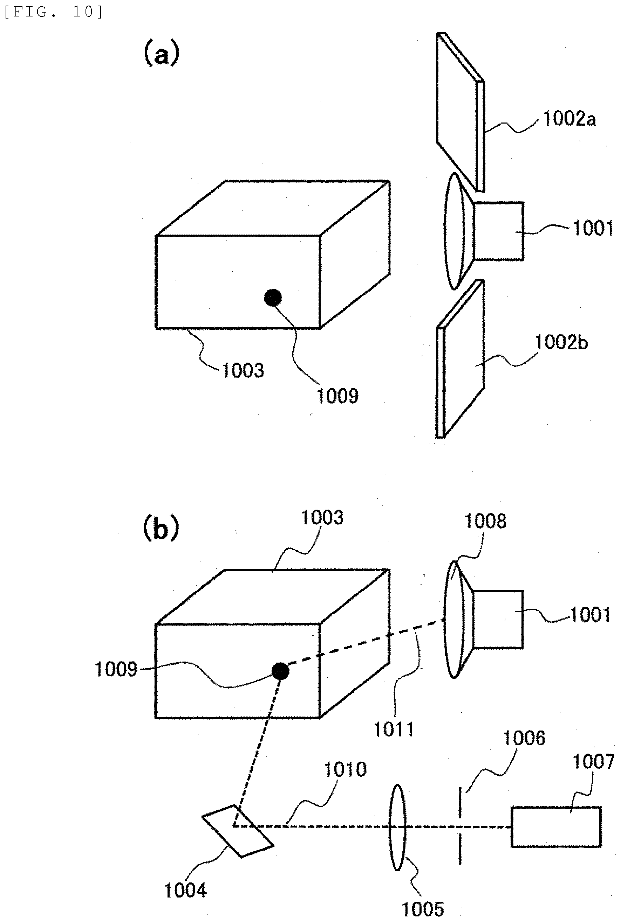

Next, the locations of the introduced cells are determined (step S802) . Here, Fig. 10 illustrates an example of determining locations of cells by using a camera according to the present embodiment. In the method illustrated in Fig. 10 (a), by using a camera 1001 and a lighting plate 1002, light reflected by the lighting plate 1002 is captured by using the camera 1001, and thus the relative locations of cells 1009 with respect to the culture vessel 1003 can be measured. Here, in order to minimize the influence of halation between a surface of the culture vessel 1003 and the camera 1001, it is desirable to use two lighting plates 1002a and 1002b as illustrated in Fig. 10(a).

In addition, in the method illustrated in Fig. 10(b), in order to determine locations of cells, the cells 1009 are stained with a fluorescent dye, a monochromatic light source 1007 such as a laser is used as excitation light in the culture vessel 1003, and excitation light source is scanned in the culture vessel 1003 by using a movable mirror 1004. Therefore, the locations of the cell 1009 emitting fluorescent light can be determined with the camera 1001. In this method, since light source is three-dimensionally scanned by using the movable mirror 1004, locations of cells can be effectively determined by using an appropriate pinhole 1006 and a lens 1005 to make an excitation light source as a confocal system.

In the methods illustrated in Figs. 10(a) and 10(b), since it is necessary to determine locations of cells in the three-dimensional space inside the culture vessel 1003, it is effective to install the cameras 1001 in three directions such as the X-axis direction, the Y-axis direction, and the Z-axis direction for more accurate determination of the locations of cells. Furthermore, when relative locations of the camera 1001 and the culture vessel 1003 are changed, fluctuation of locations of cells can be minimized by performing a calibration of relative positions. Specifically, for example, by using a calibrator that determines absolute positions with an inactive substance during a cell culturing process in the culture vessel 1003, a correction coefficient is determined based on an amount of divergence from true values of the values measured by the camera 1001 of the calibrator, and calibration of the location of the camera 1001 can be performed.

Next, cells are manipulated by applying a magnetic field (step S803), and locations of the cells are measured by using the camera 1001 (step S804). Next, it is determined whether the measured locations of the cells are identical to target locations (step S805).

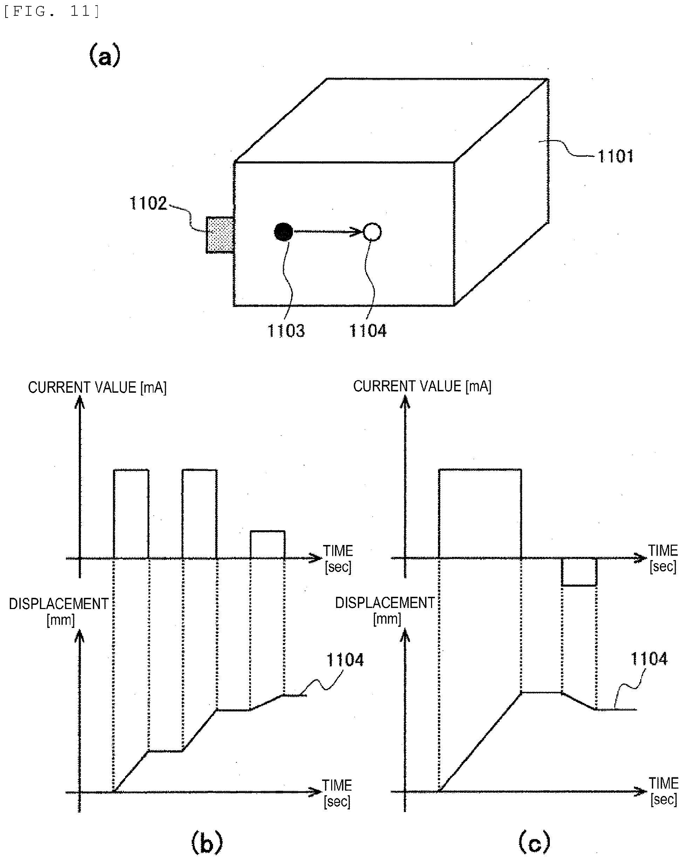

Here, Fig. 11 illustrates a specific example of a magnetic field formation pattern according to the present embodiment. Fig. 11(a) illustrates an example of an operation for moving a cell 1103 in a culture vessel 1101 to a destination point 1104 by using a magnetic field generated by a magnetic field generator 1102 outside the culture vessel 1101. In general, the magnetic field generator 1102 employs the electromagnet 201 capable of freely controlling a magnetic field using an electric current, and a magnetic field H generated by a representative solenoid coil therein may be expressed by an Equation (1) : H = n · I (n: the number of times coils is wound, I: a current value). Subsequently, a force F[N] generated when a magnetic pole of m[Wb] is arranged in the magnetic field H can be expressed as (Equation 2): F = m · H.

The two equations stated above can be combined with each other and expressed as (Equation 3) : F = m · n · I, and a cell incorporating a magnetic substance of m[Wb] can apply an external force based on a current value. Next, when a force of F[N] is applied to a material having a mass of M[kg], an acceleration a[m/s^2] can be expressed as (Equation 4): a = F/M. Then, a displacement x of an object moving with the acceleration a[m/s^2] after t[s] at the initial velocity of 0 can be expressed as (Equation 5) : x = (1/2) · a · (t)^2, based on (Equation 3), (Equation 4), and (Equation 5), (Equation 6) can be expressed as x = (m · n/2M) · I · t^2. Here, according to (Equation 6), not only displacement of cells incorporating a magnetic substance can be controlled with a current value and an application time, but also an optimum current value and an optimum application time until cells arrive at destination points can be calculated.

Furthermore, as illustrated in Fig. 11(b), cells are controlled by an external magnetic field for a certain distance by applying a current in a pulsed manner (step S803), locations of the cells are checked with the camera 1001 (step S804), the cells can be manipulated again based on the measured locations of the cells (step S805). Furthermore, in step S804, if it is determined based on a result of image captured by the camera 1001 that a moving distance per pulse at the current value used until now is too large with respect to a distance to the destination point 1004, conditions can be adjusted to lower the current value for next cell movement, and thus cells can be manipulated with smaller moving distance per pulse. Therefore, the cells can be reliably moved to the destination point 1004.

Next, in step S804, as illustrated in Fig. 11(c), if it is determined that a movement amount per pulse passed the destination point 1004, the current value applied to the solenoid coil may be reversed in a next cell manipulation, thereby reversing a direction of the magnetic field and moving the cells to the destination point 1004. Since it is necessary to manipulate the cells 1103 incorporating a magnetic substance three-dimensionally inside of the culture vessel 1101 as in the present embodiment, it is preferable to install the magnetic field generators 1102 in three directions such as the X-axis direction, the Y-axis direction, and the Z-axis direction for more precise cell manipulation.

Next, the cells introduced into the culture vessel 103 are cultured and fixed (step S806).

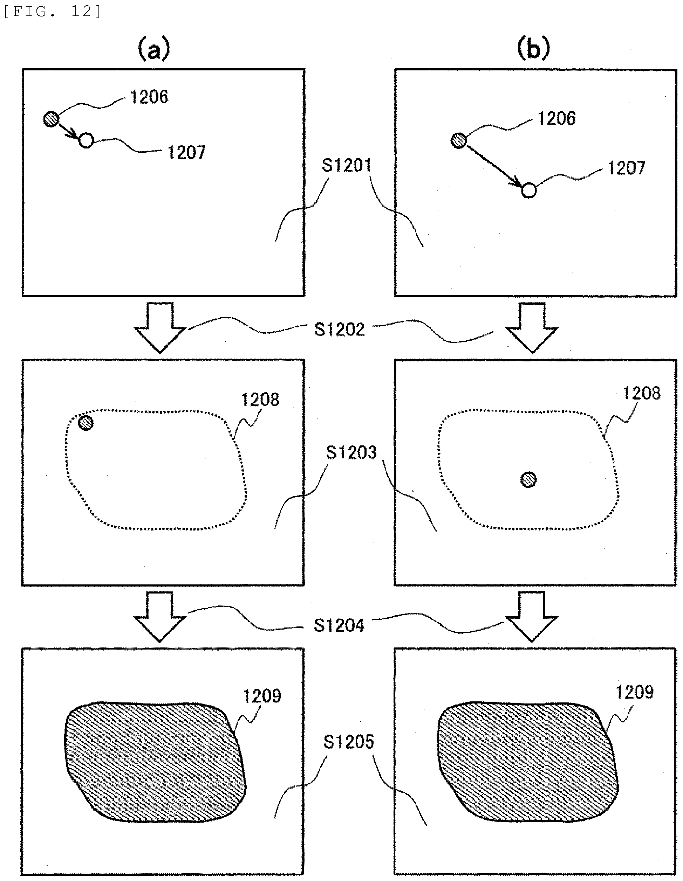

Here, Fig. 12 illustrates the state (a) at the beginning of culturing (before fixed) and the state (b) after culturing (fixed) of single layer or multilayer cells according to the present embodiment. In the present embodiment, when cells are manipulated one by one, either the method illustrated in Fig. 12(a) of fixing cells from edges or the method illustrated in Fig. 12 (b) of fixing cells from the center with respect to the target shape 1208 of a cell mass may be used.

The method illustrated in Fig. 12 (a) is useful for cases such as tissue regeneration for a simple tissue where the highest priority is given to the shape thereof. Also, the method illustrated in Fig. 12(b) is useful for cases such as organ regeneration when the highest priority is given to internal functions of internal organs. In any of the methods illustrated in Figs. 12(a) and 12(b), as an order for fixing cells, single layer cell sheets can be formed by one cell and a next single layer cell sheet can be formed thereon as described above in the Embodiment 1. Alternatively, a three-dimensional cell mass can be formed as a core first and cells can be formed around the core. It is desirable to employ an optimal method according to conditions of a three-dimensional structure from cells to be fabricated.

### Embodiment 3

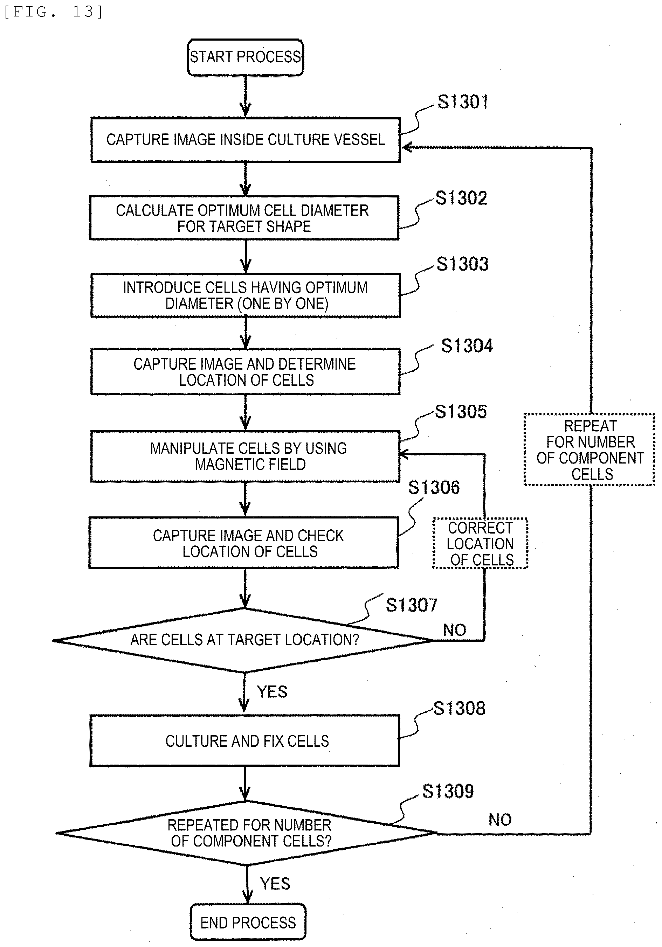

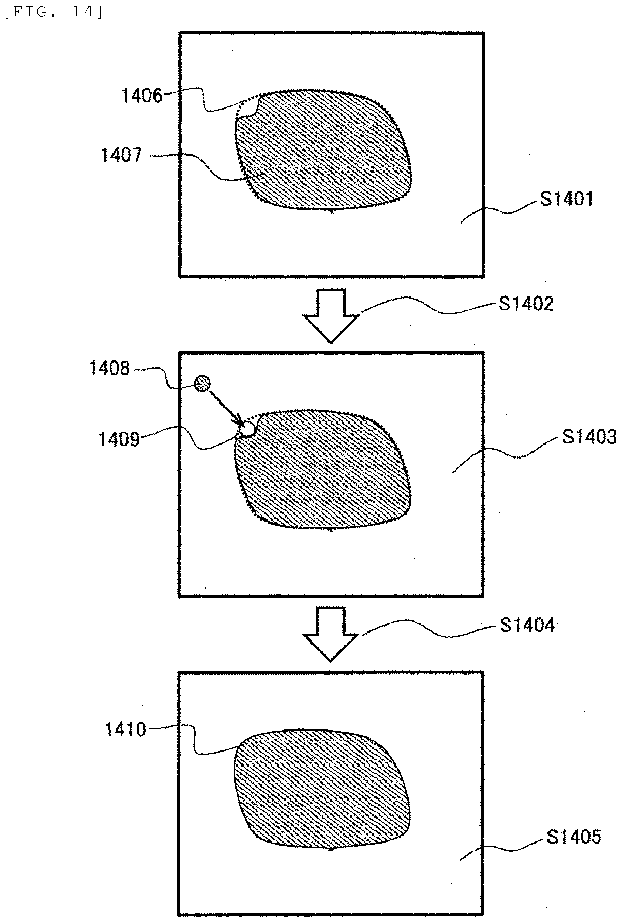

In Embodiment 1 and Embodiment 2 described above, examples of fabrication of three-dimensional structures from cells where the diameter of used cells is uniform are illustrated. However, in order to improve the reproducibility of the shape of an organ formed by a three-dimensional structure from cells, it is sometimes necessary to use several cells with different diameters. In the present embodiment, fabrication of a three-dimensional structure from a plurality of cells having different diameters will be described with reference to Figs. 13 and 14.

Fig. 13 is a flowchart illustrating basic operation for fabricating a three-dimensional structure from a plurality of cells having different diameters according to the present embodiment, and Fig. 14 is a diagram illustrating states from the beginning of culturing to fixation of cells according to the present embodiment.

First, in step S1401, the interior image of the culture vessel 103 is captured (step S1301) and, from a result of the image capturing, a target shape of a cell mass and an image of a cell mass fixed until previous steps are extracted from the result of the image capturing. Based on the extracted result, a cell diameter necessary for a next step is calculated (step S1302).

Next, in step S1402, cells having the cell diameter determined in step S1302 are introduced into the culture vessel 103. Next, in step S1403, the interior image of the culture vessel 103 is captured (step S1304) and, as in the Embodiment 2, image capturing with the camera 1001 and manipulation of cells using a magnetic field are repeatedly performed(steps S1304, S1305, and S1306), thereby manipulating the cells to target locations and fixing the cells thereto. As a result, a cell mass is formed.

According to the present embodiment, it is possible to significantly reduce the time required to form a cell mass by using cells having a large diameter for a part that includes cells of a same type and occupies a large volume. Also, by using cells having small diameters for a part with a complicated shape, shape reproducibility of the cell mass can be improved.

### Embodiment 4

In the above-described embodiments, a three-dimensional cell structure is fabricated without removing magnetic substances from magnetized cells. Depending on types of cells and magnetic substances, an incorporated magnetic substance can be naturally discharged by the metabolism of the cells. In such a case, even if a process for removing the magnetic substances is not performed, it is thought that there is no influence from the magnetic substances. However, depending on types of cells, types of magnetic substances, and sizes of cells, natural discharge of a magnetic substance may be insufficient. Also, depending on purposes of a cell mass, it may be necessary to remove a magnetic substance reliably when the cell mass is used for transplantation into a body, for example, clinical use. In such a case, it is necessary to reliably remove a magnetic substance in cells through an external manipulation.

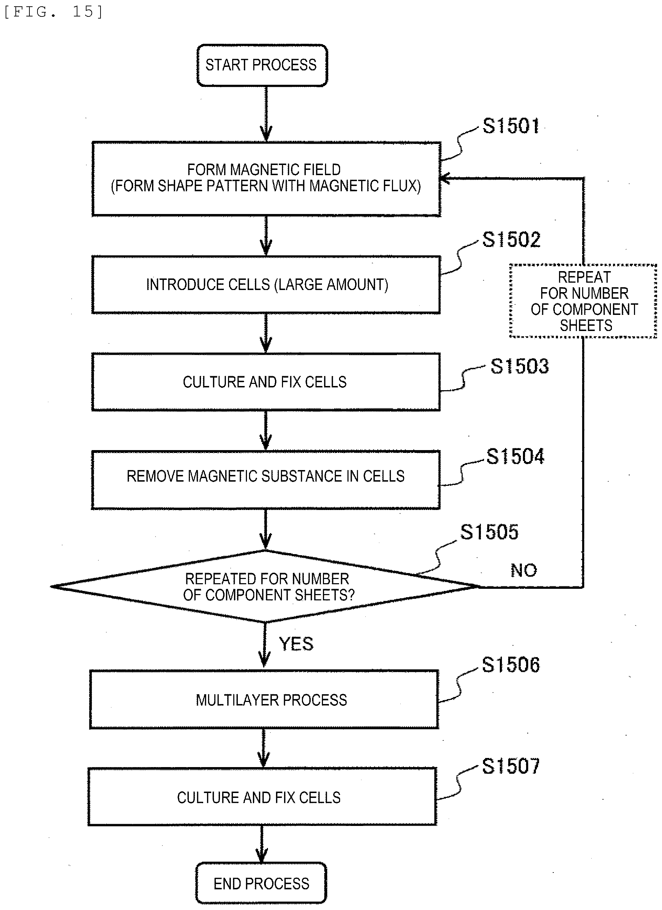

In the present embodiment, embodiments in which a process for removing a magnetic substance in a cell is included after formation of a three-dimensional structure by using cells incorporating the magnetic substance according to the Embodiments 1 and 2 will be described with reference to Figs. 15 to 17, and Fig. 21.

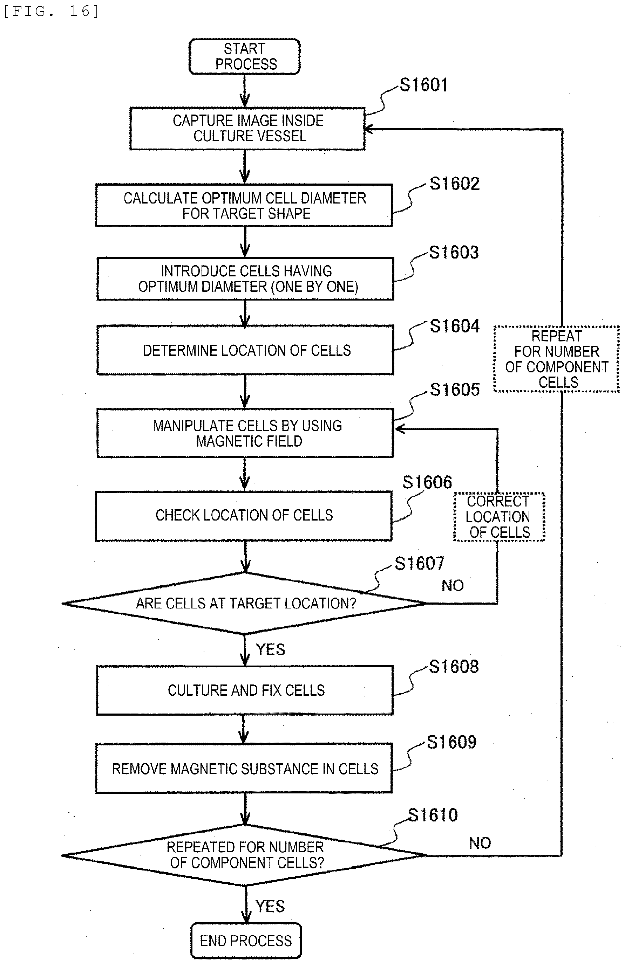

Figs. 15 and 16 are flowcharts illustrating basic operations of embodiments in which a process for removing magnetic beads according to the present embodiment is added to the fabrications of three-dimensional structures from cells described above in the Embodiments 1 and 2, respectively. In the flowchart of Fig. 15, after formation of a single layer cell sheet (step S1503), a magnetic substance removal operation (step S1504) is performed. Also, in the flowchart of Fig. 16, after fixation of one cell (step S1608), a magnetic substance removal operation (step S1609) is performed.

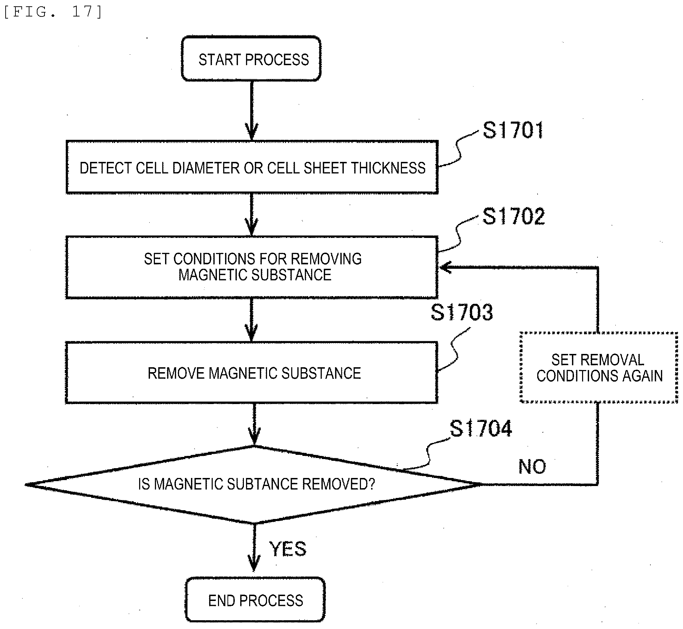

Fig. 17 is a flowchart illustrating details of a magnetic bead removing process according to the present embodiment.

First, the thickness of a cell sheet or the diameter of cells is measured by the camera 1001. In case of applying the Embodiment 1, the thickness of the cell sheet is measured. In case of applying the Embodiment 2, the diameter of the cells is measured (step S1701). During an operation for removing the magnetic substances, when the thickness of the cell sheet or the diameter of cells is large, if a metabolic activator or the like is applied for promoting discharge of the magnetic substances, it is difficult for the activator to reach the cells in the center portion of the cell sheet. Therefore, by acquiring such information in the present step, various conditions (temperature, time, and the like) for reliably transferring the activators to the center portion can be optimally set.

The conditions (temperature, time, and the like) for removing a magnetic substance are acquired (step S1702), considering the type of the cells and the magnetic substances, based on the information including the diameter of the cells or the thickness of a cell sheet measured in step S1701.

Next, by using the magnetic substance removal conditions determined in step S1702, the metabolic activator or the like is applied to the cells, thereby removing the magnetic substance (step S1703).

Thereafter, in step S1704, it is determined whether the magnetic substance is reliably removed through the removal process in step S1703 (step S1704). Specifically, several single cells are arranged as controls in the culture vessel 103, and locations of the cells are measured with the camera 1001 when an external magnetic field is generated by the magnetic field generator 104 with respect to the controls. Therefore, it may be determined whether the magnetic substance is removed. By performing the above removal process, it becomes possible to reliably remove a magnetic substance and to fabricate a three-dimensional structure guaranteeing high safety in any circumstances.

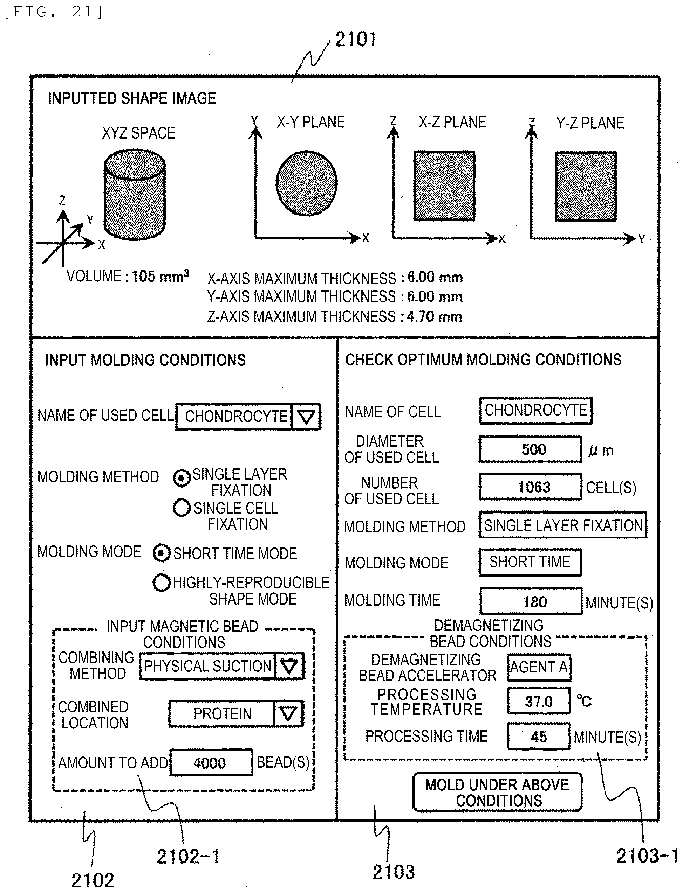

Here, Fig. 21 is a diagram illustrating a GUI screen displaying information about a three-dimensional structure from cells according to the present embodiment.

In Fig. 21, a shape image display unit 2101, a molding condition input unit 2102, and an optimum molding condition display unit 2103 are the same as the components described above with reference to Fig. 20, respectively. However, since the present embodiment includes a process for removing a magnetic substance, a magnetic bead condition input unit 2012-1 for inputting information necessary for setting conditions for the removal process, and a demagnetizing bead condition display unit 2103-1 for displaying required removal conditions based on the input information are further provided.

As described above, according to the present embodiment described above, a regenerated tissue/regenerated organ having a desired size, shape, or structure can be formed by adjusting culture conditions with high precision and without causing contamination or damage to cells.

It should be noted that the present invention is not limited to the above embodiments, but includes various modified examples. For example, the above embodiments have been described in detail in order to explain the present invention in an easy-to-understand manner, and are not necessarily limited to those having all the configurations described. Further, a part of the configuration of one embodiment can be replaced with the configuration of another embodiment, and the configuration of another embodiment can be added to the configuration of one embodiment. Further, it is possible to add, delete, and replace other configurations with respect to a part of configuration of each embodiment.

### Reference Signs List

101: incubator
102: arm
103: culture vessel
104: magnetic field generator
105: camera
106: magnetic field processing engine
107: arm processing engine
108: image processing engine
109: data bus
110: control unit
111: display unit
112: input unit
201: electromagnet
202: X-axis movement adjusting mechanism
203: Y-axis movement adjusting mechanism
401: external magnetic field generator
402: target shape of cell mass (shape pattern)
403: magnetic field application pattern
501: arm
502: well plate
503: culture vessel
504: centrifuge tube
505: tube
506: valve
507: cell
508: nozzle
601: cells arranged as a single layer
602: cell sheet formed after culturing
709: single layer cell sheet arranged in multiple layers
710: cell mass formed after culturing
711: cell layer arranged on single layer sheet via magnetic field
712: single layer cell sheet
713: cell sheets for two layers
901: arm
902: well plate
903: culture vessel
904: cell
905: tube
906: valve
907: nozzle
1001: camera
1002: lighting plate
1003: culture vessel
1004: movable mirror
1005: lens
1006: pinhole
1007: monochromatic light source
1008: color filter
1009: cell
1010: excitation light beam
1011: fluorescent light beam
1101: culture vessel
1102: magnetic field generator
1103: cell incorporating magnetic substance
1104: destination of cell manipulation
1206: cell incorporating magnetic substance
1207: destination of cell manipulation
1208: target shape of cell mass
1209: formed cell mass
1801: three-dimensional cell structure information input unit
1802: fabrication condition acquiring unit
1803: mechanism control unit
1804: image processing engine
1805: arm processing engine
1806: magnetic field processing engine
1807: database
1901: input shape image on X-Z plane
1902: input shape image on Y-Z plane
1903: input shape image on X-Y plane
1904: input shape image in XYZ space
2001, 2101: shape image display unit
2002, 2102: molding condition input unit
2102-1: magnetic bead condition input unit
2003, 2103: optimum molding condition display unit
2103-1: demagnetizing bead condition display unit

## Claims

1. A device for forming a three-dimensional structure for fabricating a three-dimensional structure of cells, the device comprising:
a culture vessel in which magnetized cells are arranged;
a magnetic field generator configured to generate a magnetic field in the culture vessel;
an incubator configured to accommodate the culture vessel therein and maintain the interior of the culture vessel at a predetermined temperature;
a control unit configured to control culture conditions of cells arranged in the culture vessel; and
an input unit through which information about a three-dimensional structure to be fabricated is input from an operator,
the control unit being configured to acquire fabrication condition for the corresponding three-dimensional structure based on the input information, and
the control unit being configured to control the magnetic field generator to generate a magnetic field based on the acquired fabrication conditions.

2. The device for forming a three-dimensional structure according to claim 1, wherein
the magnetic field generator includes a plurality of electromagnets,
the control unit is configured to acquire a shape pattern of the corresponding three-dimensional structure based on the input information, and
based on the acquired shape pattern, the control unit is configured to control electromagnets arranged within a predetermined region from among the plurality of electromagnets in the corresponding magnetic field generator to selectively generate a magnetic field.

3. The device for forming a three-dimensional structure according to claim 1, wherein
the magnetic field generator includes an electromagnet for generating a magnetic field and a moving mechanism for moving the location of the electromagnet,
the control unit is configured to set target locations to arrange magnetized cells based on the input information, and
the control unit is configured to control the electromagnet to change its location based on the set target locations.

4. The device for forming a three-dimensional structure according to claim 3, further comprising:
an image capturing unit configured to capture images of cells arranged in the culture vessel, wherein
the control unit controls the electromagnet to move, such that locations of the cells are brought close to the target locations based on the information acquired through the image capturing unit.

5. The device for forming a three-dimensional structure according to claim 1, further comprising:
a display unit configured to display fabrication conditions acquired by the control unit.

6. The device for forming a three-dimensional structure according to claim 1, wherein
the control unit acquires a shape image of a three-dimensional structure to be fabricated based on the input information, and
the device further includes a display unit configured to display the shape image acquired by the control unit.

7. The device for forming a three-dimensional structure according to claim 1, wherein
the input unit is capable of receiving an input of information about a magnetic substance used in the corresponding magnetized cells, and
the control unit acquires conditions related to removal of the magnetic substance during fabrication of the corresponding three-dimensional structure based on the corresponding input information about the magnetic substance.

8. The device for forming a three-dimensional structure according to claim 1, further comprising:
an arm configured to introduce magnetized cells into the culture vessel, wherein
the control unit controls the operation of the arm so as to introduce the cells based on the acquired fabrication conditions.

9. The device for forming a three-dimensional structure according to claim 6, wherein
the display unit displays a shape image of the three-dimensional structure to be fabricated among at least any of an X-Y plane, an X-Z plane, a Y-Z plane, and an XYZ space.

10. A method of fabricating a three-dimensional structure from cells, the method comprising:
acquiring fabrication conditions of a three-dimensional structure to be fabricated based on externally input information about the three-dimensional structure;
based on the acquired fabrication conditions, generating a magnetic field in a culture vessel for culturing cells;
introducing magnetized cells into the culture vessel in which the magnetic field is generated; and
culturing the introduced cells.

11. The method of fabricating a three-dimensional structure from cells according to claim 10, wherein
the fabrication conditions of the three-dimensional structure include a shape pattern of the three-dimensional structure, and
based on the acquired shape pattern, a magnetic field is selectively generated in a predetermined region of the culture vessel for culturing cells.

12. The method of fabricating a three-dimensional structure from cells according to claim 10, wherein
the fabrication conditions of the three-dimensional structure include target locations in the culture vessel to arrange the introduced magnetized cell, and
based on the acquired target locations, a generation location of a magnetic field in the culture vessel are set to be movable.

13. The method of fabricating a three-dimensional structure from cells according to claim 12, further comprising:
capturing an image of cells arranged in the culture vessel, wherein
the generation location of the magnetic field is moved so as to bring the locations of the cells close to the target locations based on the captured image information.

14. The method of fabricating a three-dimensional structure from cells according to claim 10, wherein
the information about the three-dimensional structure to be fabricated includes information about a magnetic substance used in the magnetized cells, and
the method further includes acquiring conditions related to removal of the magnetic substance during fabrication of the corresponding three-dimensional structure based on the information about the magnetic substance and removing the magnetic substance based on the acquired conditions.
